# FASCICULE DE BREVET EUROPEEN

(11) **EP 3 786 965 B1**
(45) Date de publication et mention de la délivrance du brevet: **19.03.2025**
(21) Numéro de dépôt: 20188155.4
(22) Date de dépôt: 28.07.2020
(51) Int. Cl.: G16H 10/40, B01L 9/06

(54) **EQUIPEMENT ET PROCÉDÉ POUR LA GESTION AUTOMATISÉE DE POTS D'ÉCHANTILLONS BIOLOGIQUES**
AUSSTATTUNG UND VERFAHREN ZUR AUTOMATISCHEN VERWALTUNG VON BECHERN MIT BIOLOGISCHEN PROBEN
DEVICE AND METHOD FOR AUTOMATED MANAGEMENT OF CONTAINERS OF BIOLOGICAL SAMPLES

(30) Priorité: 30.08.2019 FR 1909562
(43) Date de publication de la demande: 03.03.2021
(73) Titulaire: Dreampath Diagnostics, 67100 Strasbourg (FR)
(72) Inventeur: JORDAN NAVAS, Pablo, 28005 MADRID (ES); WILHELM, Valérie, 67114 ESCHAU (FR)
(74) Mandataire: Hugues, Catherine

(56) Documents cités:
- EP-A1- 3 182 310
- WO-A1-2015/040320
- US-A1- 2016 210 524

## Description

La présente invention a pour objet un équipement et un procédé pour la gestion automatisée d'une pluralité de prélèvements biologiques placés chacun dans un pot d'échantillonnage en vue d'analyses destinées à l'établissement d'un diagnostic.

De manière classique, les laboratoires de pathologie réceptionnent chaque jour des échantillons biologiques fraîchement prélevés, placés dans des pots de formol de différentes tailles, qu'ils enregistrent dans leur système informatique avant de les soumettre à différentes méthodes d'analyse. Ces dernières sont sélectionnées cas par cas et impliquent couramment un véritable parcours de ces échantillons biologiques au sein des laboratoires, au cours duquel ils sont manipulés par différents spécialistes ou techniciens et par conséquent déplacés à maintes reprises.

En pratique, dans le meilleur des cas, des analyses effectuées en une seule étape à partir de l'intégralité de la matière prélevée auprès d'un même patient, et par conséquent l'intégralité de la matière contenue dans un même pot, peuvent conduire à l'établissement d'un diagnostic fiable.

Toutefois, une ou plusieurs étapes consistant à étudier la matière, fraction après fraction, selon différentes méthodes, sont souvent nécessaires pour obtenir un diagnostic considéré comme satisfaisant.

Typiquement, les pièces opératoires de grande dimension, pour lesquelles certaines parties seulement semblent a priori pertinentes pour l'établissement d'un diagnostic, peuvent être concernées par un tel traitement. De fait, des analyses complémentaires, menées à partir de nouvelles fractions, se révèlent régulièrement nécessaires, et supposent par conséquent de retrouver le pot initial dans lequel la partie résiduelle est conservée pour poursuivre les investigations.

Quoiqu'il en soit, la règlementation en vigueur contraint les laboratoires de pathologie à conserver toute fraction initialement écartée du processus d'analyse, dans le pot d'échantillonnage d'origine, jusqu'à l'établissement définitif du diagnostic, pour être en mesure d'y recourir dans un second temps, et d'effectuer d'éventuelles analyses complémentaires.

Par ailleurs, dans certains cas, des examens cytologiques secondaires s'avèrent nécessaires pour confirmer ou infirmer des résultats de tests primaires, et supposent également un stockage, jusqu'au résultat final, du prélèvement biologique considéré dans son pot de formol d'origine.

Les prélèvements biologiques de type biopsies sont, quant à eux, généralement extraits tels quels dans leur intégralité de leur pot de formol en vue d'une intégration dans un bloc de paraffine permettant ensuite de réaliser des lames pour l'observation microscopique. Mais il est à noter que certains pots sont susceptibles de contenir plusieurs biopsies prélevées sur un même patient, pouvant donner lieu à différents blocs de paraffine fabriqués de manière échelonnée dans le temps. De telles biopsies doivent également demeurer maintenues dans le formol du pot d'échantillonnage initial jusqu'à la réalisation de l'ensemble des blocs.

Il ressort de ce qui précède que les laboratoires de pathologie sont confrontés à une première contrainte d'organisation, supposant la possibilité de localiser rapidement et de manière fiable un prélèvement biologique, et par conséquent le pot d'échantillonnage dans lequel il est conservé, pour effectuer ou finaliser des diagnostics et proposer des traitements adaptés, tout en épargnant les patients de nouveaux prélèvements intempestifs occasionnés par une éventuelle perte de matière, et susceptibles de générer un stress important.

D'autre part, dans tous les cas, après une période de rétention obligatoire, qui s'achève classiquement deux à quatre semaines après la publication du compte-rendu de diagnostic, les pots d'échantillonnage et les éventuels résidus de matière biologique qu'ils contiennent encore peuvent être détruits, ce qui permet notamment de libérer de l'espace pour accueillir les nouveaux pots et travailler dans un environnement le mieux organisé possible.

Être en mesure d'identifier et de localiser rapidement, de manière fiable, les pots d'échantillonnage à détruire représente un second défi pour les laboratoires de pathologie, qui ne disposent actuellement d'aucune solution rationnelle ni d'aucun matériel spécifiquement étudié pour parvenir à cet objectif. De fait, de manière classique, les pots présents au sein des laboratoires sont simplement rangés pêle-mêle dans des paniers, permettant leur transport de manière regroupée, et y demeurent tout au long du processus. Au terme de celui-ci, un tri manuel, effectué à l'aide d'une liste générée via le système informatique des laboratoires, s'impose généralement permettant d'extraire de ces paniers les pots d'échantillonnage pour lesquels la destruction est autorisée. De toute évidence, une telle manière de procéder s'avère fastidieuse et n'exclut nullement le risque d'erreur.

En somme, à l'heure actuelle, les laboratoires de pathologie subissent une perte de temps et d'efficacité considérable dans la mesure où ils ne disposent d'aucune solution rationnelle de gestion des pots d'échantillons biologiques dont ils ont la charge. Une aggravation de cette situation est par ailleurs à craindre dans la mesure où le nombre de pots à traiter est en augmentation constante en raison, entre autres, du vieillissement de la population et de l'augmentation de l'incidence de certaines maladies telles que notamment le cancer.

L'objet de la présente invention est de proposer un équipement et un procédé spécifiquement étudiés pour permettre une gestion rationnelle des échantillons biologiques et des pots les contenant par les laboratoires de pathologie ou par tout service équivalent en charge de la réalisation d'analyses et de l'établissement de diagnostics. Plus précisément, un objectif de l'invention est de proposer un équipement et un procédé permettant de garantir la traçabilité des échantillons biologiques contenus dans des pots d'échantillonnage, pendant toute leur durée de stockage dans de tels pots, et de procéder rapidement et de manière automatisée à leur extraction de leur lieu de stockage puis à leur destruction au moment opportun sans risque d'erreur et de pertes de prélèvements pouvant s'avérer préjudiciables pour les patients.

A cet effet, la présente invention concerne un équipement pour la gestion automatisée d'une pluralité de prélèvements biologiques placés chacun dans un pot d'échantillonnage selon la revendication 1.

Conformément à une première caractéristique de l'invention, les moyens d'entraînement en rotation du réceptacle sont définis par un arbre monté en rotation dans la cavité dudit appareil tandis que le réceptacle consiste en un plateau présentant une face inférieure et une face supérieure comportant lesdits logements, ledit arbre et ledit plateau présentant des moyens d'assemblage temporaire du plateau audit arbre, conçus aptes à bloquer en rotation le plateau par rapport audit arbre.

Selon une variante de réalisation envisageable, les moyens d'assemblage comportent un embout de section polygonale prolongeant une extrémité libre de l'arbre et un orifice de forme complémentaire à la section de l'embout s'étendant depuis la face inférieure du plateau à travers au moins une partie de l'épaisseur de ce dernier.

Par ailleurs, en vue de disposer d'un réceptacle d'usage universel, il a été imaginé d'équiper celui-ci de logements présentant des formes et des dimensions variables l'un par rapport à l'autre, et pouvant par conséquent accueillir simultanément des pots de différentes formes et dimensions.

Avantageusement, lesdits logements et/ou lesdits pots d'échantillonnage peuvent en outre comporter des moyens détrompeurs permettant d'assurer un positionnement convenable d'un pot dans un logement. Une telle caractéristique permet de limiter les éventuels problèmes ou défauts de lecture des données encodées par les moyens de lecture dont l'appareil est équipé, grâce à une orientation adéquate des pots en direction desdits moyens de lecture.

L'équipement selon l'invention est encore caractérisé en ce qu'il comporte un lecteur de données encodées additionnel relié auxdits moyens de traitement informatiques. En l'occurrence, il a été imaginé de compléter l'équipement d'un dispositif additionnel de lecture de données encodées, de type notamment « douchette », permettant une vérification rapide du contenu d'un pot d'échantillonnage stocké dans un moyen de rangement donné. Une telle douchette peut en outre présenter un écran d'affichage permettant à un opérateur d'y visualiser directement les résultats de ses vérifications.

De plus, conformément à l'invention, l'équipement peut encore comporter au moins un élément de mobilier conçu apte à héberger une pluralité de réceptacles.

Par ailleurs, il a également été imaginé que le réceptacle dont est pourvu l'équipement selon l'invention peut comporter des moyens de signalisation de la position de pots pour lesquels un événement donné est détecté, lesdits moyens de signalisation étant reliés aux moyens de traitement informatique.

Une caractéristique additionnelle de l'équipement selon l'invention est définie par le fait qu'il peut comporter des moyens d'extraction automatique dudit réceptacle de pots pour lesquels un événement donné est détecté, lesdits moyens d'extraction automatique étant reliés aux moyens de traitement informatique.

L'invention a également pour objet un procédé de gestion automatisée d'une pluralité de prélèvements biologiques placés chacun dans un pot d'échantillonnage selon la revendication 10.

Par ailleurs, une caractéristique additionnelle consiste en ce que lors de la détection dudit événement donné, on déclenche des moyens d'extraction automatique des pots du réceptacle que comporte l'équipement.

La présente invention sera davantage détaillée dans la description qui suit, faite en référence à la figure annexée, fournie à titre d'exemple non limitatif, et illustrant une vue simplifiée d'une variante de réalisation de l'équipement selon l'invention.

Comme indiqué ci-dessus, la présente invention a pour objet un équipement 1 pour la gestion automatisée d'une pluralité de prélèvements biologiques 2 placés chacun dans un pot d'échantillonnage 3a, 3b, 3c, etc., généralement de forme tubulaire, comportant un fond 30 surmonté par une paroi périphérique 31 et fermé par un couvercle 32.

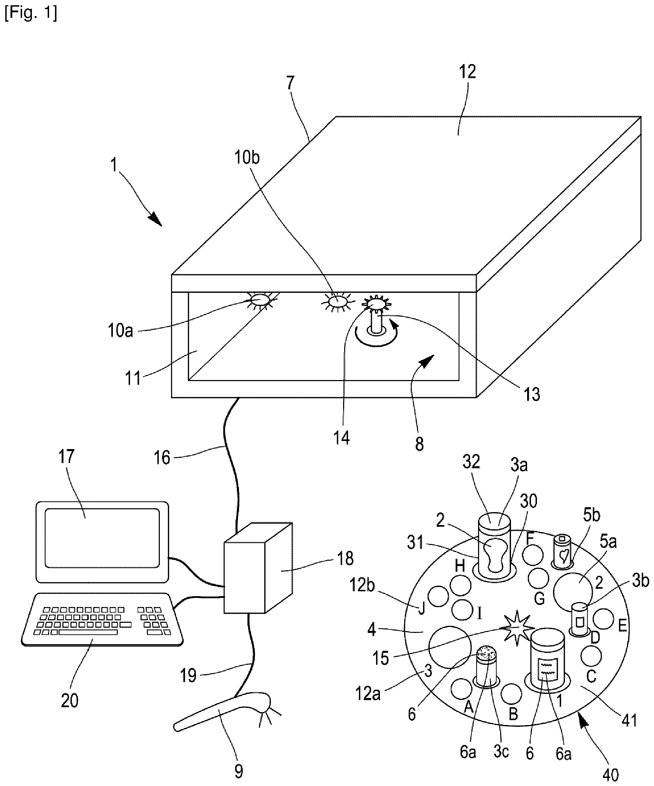

Tel qu'illustré à la figure 1, l'équipement 1 comporte au moins un réceptacle 4 de stockage de pots d'échantillonnage 3a, 3b, 3c, etc., défini ici par un plateau présentant une face inférieure 40, et une face supérieure 41 dans laquelle une pluralité de logements sous forme de creux 5a, 5b, 5c, etc., de forme complémentaire à un fond 30 d'un pot d'échantillonnage 3a, 3b, 3c, etc., ont été ménagés. De manière classique, après introduction d'un échantillon biologique 2 dans un pot 3a, 3b, 3c, etc., des données d'identification encodées, par exemple sous forme de code-barres ou code matriciel 6a ou puce RFID, sont apposées directement sur la paroi périphérique 31 ou le couvercle 32 de chaque pot, ou au moyen d'une étiquette adhésive 6 collée sur l'un et/ou l'autre de ces mêmes emplacements. Il est à noter que dans la variante de réalisation illustrée, les logements 5a, 5b, 5c, etc., présentent des formes et des dimensions variables l'un par rapport à l'autre, ce qui permet de stocker sur un même réceptacle 4 des pots d'échantillonnage 3a, 3b, 3c, etc., de formes et de dimensions variables.

L'équipement 1 comporte en outre au moins un appareil 7 se présentant sous la forme d'un boîtier, délimitant une cavité 8, conformée pour accueillir au moins un réceptacle 4. La cavité 8 est équipée de moyens de lecture 10a, 10b des données d'identification encodées apposées sur la paroi périphérique 31 et/ou le couvercle 32 des pots d'échantillonnage 3a, 3b, 3c, etc., placés dans le réceptacle 4. Ces moyens de lecture peuvent être définis par exemple par un ou plusieurs scanners lumineux 10a, 10b de données encodées, notamment de type code à barres ou code matriciel 6a, ou lecteur(s) RFID, montés de manière appropriée dans la cavité 8, par exemple sur la paroi périphérique 11 ou le couvercle 12 du boîtier délimitant la cavité 8.

De plus, l'appareil 7 comporte également des moyens de détermination de données représentatives de la position de chaque pot d'échantillonnage 3a, 3b, 3c, etc., au sein du réceptacle 4, tels que par exemple des moyens de détection de chiffres 12a ou de lettres 12b, ou toute autre forme de nature quelconque, apparaissant en relief ou en creux au voisinage immédiat de chaque logement 5a, 5b, 5c, etc. Ces moyens de détermination de données représentatives de la position de chaque pot d'échantillonnage 3a, 3b, 3c, etc., peuvent être indépendants des moyens de lecture 10a, 10b de données encodées. Toutefois, en plus de la fonction de lecture de données encodées, ces derniers peuvent être conçus de manière telle qu'ils peuvent également remplir la fonction de détection et de lecture de formes apparaissant en relief ou en creux au voisinage desdits logements 5a, 5b, 5c, etc.

Il est encore à noter que des moyens d'entraînement en rotation du réceptacle 4, définis par un arbre 13 monté en rotation dans la cavité 8 de l'appareil 7 permettent, lorsqu'un réceptacle 4 pourvu de pots 3a, 3b, 3c, etc., est inséré dans l'appareil 7, de diriger chaque pot successivement en face des moyens de lecture 10a, 10b afin que ces derniers relèvent les données encodées apposées sur le couvercle 32 ou la paroi périphérique 31 de celui-ci, et éventuellement les données 12a, 12b, représentatives de la position de chaque pot d'échantillonnage 3a, 3b, 3c, etc., sur le réceptacle 4, ou sinon également en face des moyens de détermination de données représentatives de la position de chaque pot d'échantillonnage lorsqu'ils sont indépendants des moyens de lecture 10a, 10b.

A ce propos, il peut avantageusement être prévu que les logements 5a, 5b, 5c, etc., et/ou les pots d'échantillonnage 3a, 3b, 3c, etc., comportent des moyens détrompeurs permettant d'assurer un positionnement convenable des pots 3a, 3b, 3c, etc., dans les logements 5a, 5b, 5c, etc., et ce en termes d'orientation des données encodées en direction des moyens de lecture 10a, 10b des données encodées. En somme, de tels détrompeurs permettent à un opérateur en charge du positionnement des pots 3a, 3b, 3c, etc., dans le plateau 4, de disposer ceux-ci spontanément et sans tâtonnements de manière telle que l'étiquette 6 qu'ils comportent est systématiquement orientée en direction des moyens de lecture 10a, 10b lors de l'arrivée de chaque pot en face de ces derniers.

Une autre caractéristique de l'invention est définie par le fait que l'arbre 13 et le réceptacle 4 présentent des moyens d'assemblage temporaire comportant, dans l'exemple illustré, un embout 14 de section polygonale prolongeant une extrémité libre de l'arbre 13 et un orifice 15 de forme complémentaire à la section de l'embout 14 s'étendant depuis la face inférieure 40 vers la face supérieure 41 du plateau 4. Ainsi, le réceptacle 4 est fixe par rapport à l'arbre 13 et suit inévitablement le mouvement de rotation de celui-ci lors du fonctionnement de l'appareil 7.

Par ailleurs, l'équipement 1 est complété par des moyens de traitement informatique, de type ordinateur 18, reliés à l'appareil 7 par des moyens 16 de transmission de données, filaires ou non filaires, et intégrant une application logicielle exécutée pour
* enregistrer dans des moyens mémoire, pour chaque pot 3a, 3b, 3c, etc., de manière associée, les données d'identification qu'il comporte, les données 12a, 12b, représentatives de sa position au sein du réceptacle 4 et des données relatives à un historique du prélèvement biologique qu'il contient,
* détecter la survenue d'un événement donné dans l'historique de chaque pot 3a, 3b, 3c, etc., et
* générer via une interface utilisateur, telle qu'un écran 17, une liste indiquant les données d'identification des pots 3a, 3b, 3c, etc., pour lesquels l'événement est détecté et leur position 12a, 12b au sein du plateau 4.

Un lecteur 9 de données encodées additionnel, de type notamment « douchette » manuelle, relié auxdits moyens de traitement informatiques par une liaison filaire ou non filaire 19 est également prévu dans la variante de réalisation illustrée de l'équipement selon l'invention. Comme indiqué précédemment, une telle douchette peut avantageusement intégrer un écran autorisant une lecture immédiate des données relevées.

D'autre part, le plateau 4 peut également comporter des moyens de signalisation de la position de pots 3a, 3b, 3c pour lesquels un événement donné est détecté, tels que notamment des voyants lumineux et/ou des moyens d'émission de signaux sonores implantés sur le plateau 4 au voisinage de chaque logement 5a, 5b, 5c. L'activation, par les moyens de traitement informatique d'un ou plusieurs de ces moyens de signalisation permet à un opérateur de détecter immédiatement le(s) pot(s) 3a, 3b, 3c à extraire, sans avoir à consulter la liste affichée à l'écran 17 de l'ordinateur 18.

De plus, des moyens d'extraction automatique dudit plateau 4 de pots 3a, 3b, 3c pour lesquels un événement donné a été détecté peuvent encore avantageusement compléter l'équipement 1.

Avantageusement, l'équipement selon l'invention peut en outre comporter au moins un élément de mobilier, telle que par exemple un caisson ou une armoire, munis de tiroirs ou de rails, conçu apte à héberger une pluralité de plateaux 4, par exemple de manière superposée dans des tiroirs ou solidaires des rails et s'étendant alors selon une ou plusieurs colonnes. A ce propos, une variante de réalisation peut être prévue dans laquelle l'équipement comporte un bras de préhension, contrôlé par les moyens de traitement informatique, et conçu apte à extraire des pots 3a, 3b, 3c de plateaux 4, eux-mêmes stockés dans un tel élément de mobilier.

En pratique, l'équipement 1 selon l'invention permet une gestion rationnelle et automatisée de pots d'échantillonnage 3a, 3b, 3c, etc., par les services de pathologie ou d'analyses médicales compétents, au travers de la mise en œuvre d'étapes simples, efficaces et fiables consistant :
- A placer, dès leur arrivée au sein d'un laboratoire, une pluralité de pots d'échantillonnage 3a, 3b, 3c, etc., munis de données d'identification 6a apposées sur leur paroi périphérique 31 et/ou leur couvercle 32 dans les logements 5a, 5b, 5c correspondants à la forme des pots considérés, d'au moins un plateau 4,
- Pour chaque pot 3a, 3b, 3c, etc., placé dans le plateau 4, à relever et enregistrer au moyen de l'appareil 7 et des moyens de traitement informatique 18, de manière associée ses données d'identification 6a, les données 12a, 12b représentatives de sa position au sein du réceptacle 4 et des données relatives à l'historique du prélèvement biologique qu'il contient. Plus précisément, ces dernières peuvent être introduites manuellement dans les moyens de traitement informatiques 18 au moyen par exemple d'un clavier 20, et correspondent à l'ensemble des événements concernant les échantillons biologiques placés dans les pots (date de prélèvement, date(s) d'analyses, date d'émission du compte-rendu de diagnostic, etc.),

- A paramétrer l'application logicielle que comportent les moyens de traitement informatiques 18 de manière telle qu'elle détecte la survenue d'un événement donné, dans les données relatives à l'historique du prélèvement biologique contenu dans chaque pot, cet événement donné étant par exemple défini par une date t correspondant à la date t0 à laquelle un compte-rendu de diagnostic relatif à un prélèvement biologique a été émis plus une période t1, par exemple au moins égale à deux semaines.
- A récupérer sur l'écran 17 ou, le cas échéant celui de la douchette 9, une liste indiquant les données d'identification des pots pour lesquels l'événement donné est détecté et leur position au sein du réceptacle 4, ou à repérer lesdits pots sur ce dernier au travers des moyens de signalisation visuels et/ou sonores qu'il comporte,
- Et enfin à extraire du plateau 4, le cas échéant par la mise en œuvre de moyens d'extraction automatique, les pots 3a, 3b, 3c, etc., pour lesquels l'événement donné est détecté, afin de procéder à leur destruction.

En d'autres termes, l'équipement 1 selon l'invention permet de disposer à chaque instant d'une information concernant la localisation d'un prélèvement biologique donné grâce à un stockage rationnel des pots d'échantillonnage dans des réceptacles et le cas échéant dans des éléments de mobilier dédiés, et à l'enregistrement, dans le système informatique, de la position de chaque pot, associée aux données d'identification qu'il comporte au travers de l'appareil 7. Ainsi, il est possible de retrouver rapidement un prélèvement donné, simplement en interrogeant le système informatique et en localisant le cas échéant l'armoire, le plateau 4 puis l'emplacement correspondant sur ce dernier. A ce stade, une vérification, au moyen d'un scan effectué manuellement avec la douchette 9 peut être effectuée par un opérateur afin de garantir que le pot retrouvé contient bien le prélèvement biologique recherché.

Par ailleurs, l'équipement selon l'invention permet également de faciliter le tri des pots et de séparer rapidement les pots à détruire de ceux qui sont à conserver, ces derniers n'étant plus stockés pêle-mêle dans des paniers mais soigneusement rangés sur des plateaux 4 ou réceptacles comportant des logements facilement repérables et associés auxdits pots dans les moyens de traitement informatiques, capables quant à eux de générer une liste des pots contenant des prélèvements biologiques ayant donné lieu à l'établissement d'un diagnostic, par exemple au moins deux semaines avant la date à laquelle la liste est générée.

En ce qui concerne les dernières étapes du présent procédé, il convient de noter que l'invention a prévu que de manière avantageuse, lors de la détection dudit événement donné, on peut déclencher automatiquement, les moyens de signalisation de la position des pots 3a, 3b, 3c à extraire que comporte ledit réceptacle 4, permettant à un opérateur d'identifier d'un rapide coup d'œil les pots à détruire. Ceci lui épargne le recours à la liste s'affichant sur l'écran de la douchette ou de l'ordinateur.

Selon une alternative additionnelle, lors de la détection dudit événement donné, on peut également dans le cadre du présent procédé, déclencher des moyens d'extraction automatique des pots 3a, 3b, 3c du réceptacle 4 que comporte l'équipement 1, évitant ainsi toute intervention manuelle d'un opérateur.

## Revendications

1. Équipement (1) pour la gestion automatisée d'une pluralité de prélèvements biologiques (2) placés chacun dans un pot d'échantillonnage (3a, 3b, 3c) comportant un fond (30) surmonté par une paroi périphérique (31) et fermé par un couvercle (32), ledit équipement (1) comportant :
- au moins un réceptacle (4) de stockage de pots d'échantillonnage (3a, 3b, 3c) présentant une pluralité de logements (5a, 5b, 5c) de forme complémentaire à un fond (30) d'un pot d'échantillonnage (3a, 3b, 3c),
- au moins un appareil (7) se présentant sous la forme d'un boîtier délimitant une cavité (8) conformée pour accueillir au moins un réceptacle (4), et équipée de moyens de lecture (10a, 10b) de données d'identification encodées (6a, 6b) apposées sur la paroi périphérique (31) et/ou le couvercle (32) des pots d'échantillonnage (3a, 3b, 3c) placés dans ledit réceptacle (4), de moyens de détermination de données représentatives de la position de chaque pot d'échantillonnage (3a, 3b, 3c) au sein dudit réceptacle (4),
**caractérisé en ce que** le réceptacle comporte, au voisinage immédiat de chaque logement, une forme (12a, 12b) apparaissant en creux ou en relief, ladite forme (12a, 12b) définissant lesdites données représentatives de la position de chaque pot d'échantillonnage (3a, 3b, 3c) au sein dudit réceptacle (4), tandis que les moyens de détermination de données représentatives de la position de chaque pot d'échantillonnage (3a, 3b, 3c) au sein dudit réceptacle (4), dont est équipée ladite cavité dudit appareil (7), sont définis par des moyens de détection de ladite forme quelconque, ladite cavité étant également équipée de moyens d'entraînement en rotation du réceptacle (4),
et ledit équipement comportant en outre :
- des moyens de traitement informatique (18) reliés audit appareil (7) par des moyens de transmission de données (16) filaire ou non filaire et intégrant une application logicielle exécutée pour :
* enregistrer dans des moyens mémoire, pour chaque pot (3a, 3b, 3c), de manière associée, les données d'identification (6a) qu'il comporte, les données (12a, 12b) représentatives de sa position au sein du réceptacle (4) et des données relatives à l'historique du prélèvement biologique (2) qu'il contient,
* détecter la survenue d'un événement donné dans l'historique de chaque pot (3a, 3b, 3c) et générer via une interface utilisateur (17) une liste indiquant les données d'identification (6a) des pots (3a, 3b, 3c) pour lesquels l'événement est détecté et les données (12a, 12b) représentatives de la position au sein du réceptacle (4) des pots (3a, 3b, 3c) pour lesquels l'événement est détecté.

2. Equipement (1) selon la revendication 1, **caractérisé en ce que** les moyens d'entraînement en rotation du réceptacle (4) sont définis par un arbre (13) monté en rotation dans la cavité (8) dudit appareil (7) tandis que le réceptacle (4) consiste en un plateau présentant une face inférieure (40) et une face supérieure (41) comportant lesdits logements (5a, 5b, 5c), ledit arbre (13) et ledit plateau (4) présentant des moyens d'assemblage temporaire du plateau (4) audit arbre (13), conçus aptes à bloquer en rotation le plateau (4) par rapport audit arbre (13).

3. Equipement (1) selon la revendication 2, **caractérisé en ce que** les moyens d'assemblage comportent un embout (14) de section polygonale prolongeant une extrémité libre de l'arbre (13) et un orifice (15) de forme complémentaire à la section de l'embout (14) s'étendant depuis la face inférieure (40) du plateau (4) à travers au moins une partie de l'épaisseur de ce dernier.

4. Equipement (1) selon l'une quelconque des revendications précédentes, **caractérisé en ce que** lesdits logements (5a, 5b, 5c) du réceptacle (4) présentent des formes et des dimensions variables l'un par rapport à l'autre.

5. Equipement (1) selon l'une quelconque des revendications 1 à 4, **caractérisé en ce que** lesdits logements (5a, 5b, 5c) et/ou lesdits pots d'échantillonnage (3a, 3b, 3c) comportent des moyens détrompeurs permettant d'assurer un positionnement convenable d'un pot (3a, 3b, 3c) dans un logement (5a, 5b, 5c).

6. Equipement (1) selon l'une quelconque des revendications précédentes, **caractérisé en ce qu'**il comporte un lecteur (9) de données encodées additionnel relié auxdits moyens de traitement informatiques (18).

7. Equipement (1) selon l'une quelconque des revendications précédentes, **caractérisé en ce que** le réceptacle (4) comporte des moyens de signalisation de la position de pots (3a, 3b, 3c) pour lesquels un événement donné est détecté, lesdits moyens de signalisation étant reliés auxdits moyens de traitement informatique.

8. Equipement (1) selon l'une quelconque des revendications précédentes, **caractérisé en ce qu'**il comporte des moyens d'extraction automatique dudit réceptacle (4) de pots (3a, 3b, 3c) pour lesquels un événement donné est détecté, reliés auxdits moyens de traitement informatique.

9. Equipement (1) selon l'une quelconque des revendications précédentes, **caractérisé en ce qu'**il comporte au moins un élément de mobilier conçu apte à héberger une pluralité de réceptacles (4).

10. Procédé de gestion automatisée d'une pluralité de prélèvements biologiques (2) placés chacun dans un pot d'échantillonnage (3a, 3b, 3c) comportant un fond (30) surmonté par une paroi périphérique (31) et fermé par un couvercle (32) au moyen de l'équipement (1) selon l'une quelconque des revendications précédentes, dans lequel on effectue les étapes suivantes :
- On place une pluralité de pots d'échantillonnage (3a, 3b, 3c) munis de données d'identification (6a) apposées sur leur paroi périphérique (31) et/ou leur couvercle (32) dans les logements (5a, 5b, 5c) d'au moins un réceptacle (4),
- Pour chaque pot (3a, 3b, 3c) placé dans ledit réceptacle (4), on relève et on enregistre au moyen dudit appareil (7) et desdits moyens de traitement informatique (18), de manière associée ses données d'identification (6a), les données (12a, 12b) représentatives de sa position au sein du réceptacle (4) et des données relatives à l'historique du prélèvement biologique (2) qu'il contient,
- On paramètre l'application logicielle que comportent les moyens de traitement informatiques (18) de manière telle qu'elle détecte la survenue d'un événement donné dans les données relatives à l'historique du prélèvement biologique (2) contenu dans chaque pot (3a, 3b, 3c),
- On affiche automatiquement sur l'interface utilisateur (17) une liste indiquant les données d'identification (6a) des pots (3a, 3b, 3c) pour lesquels ledit événement donné est détecté et les données représentatives de la position au sein dudit réceptacle (4) de chaque pot d'échantillonnage (3a, 3b, 3c) pour lequel ledit événement donné est détecté,
- On extrait dudit réceptacle (4) les pots (3a, 3b, 3c) pour lesquels l'événement donné est détecté, et on procède à leur destruction, **caractérisé en ce que** pour relever les données représentatives de la position de chaque pot d'échantillonnage (3a, 3b, 3c) au sein dudit réceptacle (4), on détecte des formes (12a, 12b) apparaissant en creux ou en relief au voisinage de chaque logement (5a, 5b, 5c) et définissant les données représentatives de la position de chaque pot d'échantillonnage (3a, 3b, 3c) au sein dudit réceptacle (4), et on utilise des moyens de détection d'une forme quelconque, apparaissant en creux ou en relief, au voisinage immédiat de chaque logement.

11. Procédé selon la revendication précédente, **caractérisé en ce que** lors de la détection dudit événement donné, on déclenche automatiquement des moyens de signalisation de la position des pots (3a, 3b, 3c) à extraire que comporte ledit réceptacle (4).

12. Procédé selon l'une quelconque des revendications 10 ou 11, **caractérisé en ce que** lors de la détection dudit événement donné, on déclenche des moyens d'extraction automatique des pots (3a, 3b, 3c) du réceptacle (4) que comporte l'équipement (1).

## Patentansprüche

1. Ausrüstung (1) für die automatisierte Verwaltung einer Vielzahl biologischer Proben (2), die jeweils in einem Probenbehältnis (3a, 3b, 3c) platziert sind, das einen Boden (30) umfasst, auf dem sich eine Umfangswand (31) befindet und das durch einen Deckel (32) verschlossen ist, wobei die Ausrüstung (1) umfasst:
- mindestens einen Behälter (4) für eine Aufbewahrung von Probenbehältnissen (3a, 3b, 3c), der eine Vielzahl von Aufnahmen (5a, 5b, 5c) mit einer Form aufweisen, die zu einem Boden (30) eines Probenbehältnisses (3a, 3b, 3c) komplementär ist,
- mindestens eine Vorrichtung (7) in Form eines Gehäuses, das einen Hohlraum (8) begrenzt, der angepasst ist, um mindestens einen Behälter (4) aufzunehmen, und der mit Mitteln (10a, 10b) zum Lesen der codierten Identifikationsdaten (6a, 6b), die an der Umfangswand (31) und/oder dem Deckel (32) der in dem Behälter (4) platzierten Probenbehältnisse (3a, 3b, 3c) angeklebt sind, Mitteln zum Bestimmen von Daten, die für die Position jedes Probenbehältnisses (3a, 3b, 3c) innerhalb des Behälters (4) repräsentativ sind, ausgestattet ist, **dadurch gekennzeichnet, dass** der Behälter in unmittelbarer Nähe jeder Aufnahme eine hohl oder erhaben erscheinende Form (12a, 12b) umfasst, wobei die Form (12a, 12b) die Daten definiert, die für die Position jedes Probenbehältnisses (3a, 3b, 3c) innerhalb des Behälters (4) repräsentativ sind, während die Mittel zum Bestimmen von Daten, die für die Position jedes Probenbehältnisses (3a, 3b, 3c) innerhalb des Behälters (4) repräsentativ sind, mit dem der Hohlraum der Vorrichtung (7) ausgestattet ist, durch Mittel zum Erkennen der beliebigen Form definiert sind, wobei der Hohlraum auch mit Mitteln zum Indrehungversetzen des Behälters (4) ausgestattet ist und die Ausrüstung ferner umfasst:
- Datenverarbeitungsmittel (18), die durch drahtgebundene oder nicht drahtgebundene Datenübertragungsmittel (16) mit der Vorrichtung (7) verbunden sind und eine Softwareanwendung integrieren, die ausgeführt wird zum:
* Speichern in den Speichermitteln für jedes Behältnis (3a, 3b, 3c), auf zugeordnete Weise, der darin enthaltenen Identifikationsdaten (6a), die Daten (12a, 12b), die seine Position innerhalb des Behälters (4) darstellen, und Daten über den Verlauf der darin enthaltenen biologischen Probe (2) umfassen,
* Erkennen eines Eintretens eines gegebenen Ereignisses in dem Verlauf jedes Behältnisses (3a, 3b, 3c) und Erzeugen, über eine Benutzerschnittstelle (17), einer Liste, die die Identifikationsdaten (6a) der Behältnisse (3a, 3b, 3c) angibt, für die das Ereignis erkannt wird, und die Daten (12a, 12b), die die Position der Behältnisse (3a, 3b, 3c) innerhalb des Behälters (4) darstellen, für die das Ereignis erkannt wird.

2. Ausrüstung (1) nach Anspruch 1, **dadurch gekennzeichnet, dass** die Mittel zum Indrehungversetzen des Behälters (4) durch eine Welle (13) definiert sind, die in dem Hohlraum (8) der Vorrichtung (7) drehbar montiert ist, während der Behälter (4) aus einer Platte, aufweisend eine Unterseite (40) und eine Oberseite (41), besteht, umfassend die Aufnahme (5a, 5b, 5c), die Welle (13) und die Platte (4), aufweisend Mittel zum vorübergehenden Zusammenbauen der Platte (4) mit der Welle (13), die geeignet sind, um die Platte (4) relativ zu der Welle (13) zu drehverriegeln.

3. Ausrüstung (1) nach Anspruch 2, **dadurch gekennzeichnet, dass** die Mittel zum Zusammenbauen eine Spitze (14) mit polygonalem Querschnitt, die sich über ein freies Ende der Welle (13) erstreckt, und eine Öffnung (15) mit einer zu dem Querschnitt der Spitze (14) komplementären Form umfassen, die sich von der Unterseite (40) der Platte (4) mindestens über einen Teil der Dicke der Letzteren erstreckt.

4. Ausrüstung (1) nach einem der vorstehenden Ansprüche,
**dadurch gekennzeichnet, dass** die Aufnahmen (5a, 5b, 5c) des Behälters (4) relativ zueinander variable Formen und Abmessungen aufweisen.

5. Ausrüstung (1) nach einem der Ansprüche 1 bis 4,
**dadurch gekennzeichnet, dass** die Aufnahmen (5a, 5b, 5c) und/oder die Probenbehältnisse (3a, 3b, 3c) ausrichtende Mittel umfassen, die es ermöglichen, eine geeignete Positionierung eines Behältnisses (3a, 3b, 3c) in einer Aufnahme (5a, 5b, 5c) zu gewährleisten.

6. Ausrüstung (1) nach einem der vorstehenden Ansprüche,
**dadurch gekennzeichnet, dass** sie einen zusätzlichen Leser (9) für codierte Daten umfasst, der mit den Datenverarbeitungsmitteln (18) verbunden ist.

7. Ausrüstung (1) nach einem der vorstehenden Ansprüche,
**dadurch gekennzeichnet, dass** der Behälter (4) Mittel für eine Signalisierung der Position der Behältnisse (3a, 3b, 3c) umfasst, für die ein gegebenes Ereignis erkannt wurde, wobei die Signalmittel mit den Datenverarbeitungsmitteln verbunden sind.

8. Ausrüstung (1) nach einem der vorstehenden Ansprüche,
**dadurch gekennzeichnet, dass** sie Mittel für eine automatischen Entnahme der Probenbehältnisse (3a, 3b, 3c) aus dem Behälter (4) umfasst, für die ein gegebenes Ereignis erkannt wurde, die mit den Datenverarbeitungsmitteln verbunden sind.

9. Ausrüstung (1) nach einem der vorstehenden Ansprüche,
**dadurch gekennzeichnet, dass** sie mindestens ein Möbelelement umfasst, das als geeignet konzipiert ist, eine Vielzahl von Behältern (4) unterzubringen.

10. Verfahren zum automatisierten Verwalten einer Vielzahl biologischer Proben (2), die jeweils in einem Probenbehältnis (3a, 3b, 3c) platziert sind, umfassend einen Boden (30), auf dem sich eine Umfangswand (31) befindet und das durch einen Deckel (32) verschlossen ist, mittels der Ausrüstung (1) nach einem der vorstehenden Ansprüche, wobei die folgenden Schritte ausgeführt werden:
- Platzieren einer Vielzahl von Probenbehältnissen (3a, 3b, 3c), die mit Identifikationsdaten (6a) versehen sind, die an ihrer Umfangswand (31) und/oder ihrem Deckel (32) angeklebt sind, in den Aufnahmen (5a, 5b, 5c) mindestens eines Behälters (4),
- zugeordnetes Erfassen und Speichern mittels der Vorrichtung (7) und der Datenverarbeitungseinrichtung (18), für jedes Behältnis (3a, 3b, 3c), das in dem Behälter (4) platziert wird, seiner Identifikationsdaten (6a), der Daten (12a, 12b), die für seine Position innerhalb des Behälters (4) repräsentativ sind, und Daten bezüglich des Verlaufs der darin enthaltenen biologischen Probe (2),
- derartiges Parametrisieren der Softwareanwendung, die die Datenverarbeitungsmittel (18) umfassen, dass sie das Eintreten eines gegebenen Ereignisses in den Daten erkennt, die sich auf den Verlauf der in jedem Behältnis (3a, 3b, 3c) enthaltenen biologischen Probe (2) beziehen,
- automatisches Anzeigen, auf der Benutzeroberfläche (17), einer Liste, die die Identifikationsdaten (6a) der Behältnisse (3a, 3b, 3c) angibt, für die das gegebene Ereignis erkannt wurde, und der Daten, die für die Position jedes Probenahmebehältnisses (3a, 3b, 3c) innerhalb des Behälters (4), für das das gegebene Ereignis erkannt wird, repräsentativ sind,
- Extrahieren, aus dem Behälter (4), der Behältnisse (3a, 3b, 3c), für die das gegebene Ereignis erkannt wurde, und Fortfahren mit deren Zerstörung, **dadurch gekennzeichnet, dass** zum Erfassen der Daten, die für die Position jedes Probenbehältnisses (3a, 3b, 3c) innerhalb des Behälters (4) repräsentativ sind, hohl oder erhaben erscheinende Formen (12a, 12b) in der Nähe jeder Aufnahme (5a, 5b, 5c) erkannt werden und die die Daten definieren, die für die Position jedes Probenahmebehältnisses (3a, 3b, 3c) innerhalb des Behälters (4) repräsentativ sind, und Mittel zum Erkennen einer beliebigen hohl oder erhaben erscheinenden Form in unmittelbarer Nähe jeder Aufnahme verwendet werden.

11. Verfahren nach dem vorstehenden Anspruch,
**dadurch gekennzeichnet, dass** während des Erkennens des gegebenen Ereignisses automatisch Mittel zum Signalisieren der Position der zu extrahierenden Behältnisse (3a, 3b, 3c), die der Behälter (4) umfasst, ausgelöst werden.

12. Verfahren nach einem der Ansprüche 10 oder 11,
**dadurch gekennzeichnet, dass** während des Erkennens des gegebenen Ereignisses Mittel zum automatischen Extrahieren der Behältnisse (3a, 3b, 3c) aus dem Behälter (4), den die Ausrüstung (1) umfasst, ausgelöst werden.

## Claims

1. [Apparatus (1) for the automated management of a plurality of biological samples (2) each placed in a sampling jar (3a, 3b, 3c) comprising a base (30) on which a peripheral wall (31) is mounted and which is closed by a lid (32), the apparatus (1) comprising:
- at least one container (4) for storing sampling jars (3a, 3b, 3c), having a plurality of recesses (5a, 5b, 5c) which are complementary in shape to a base (30) of a sampling jar (3a, 3b, 3c),
- at least one device (7) in the form of a housing delimiting a cavity (8), which cavity is shaped to accommodate at least one container (4) and is equipped with means (10a, 10b) for reading encoded identification data (6a, 6b) affixed to the peripheral wall (31) and/or the lid (32) of the sampling jars (3a, 3b, 3c) placed in the container (4), means for determining data representative of the position of each sampling jar (3a, 3b, 3c) within the container (4), **characterized in that** the container comprises, in the immediate vicinity of each recess, a recessed or raised shape (12a, 12b), the shape (12a, 12b) defining the data representative of the position of each sampling jar (3a, 3b, 3c) within the container (4), while the means for determining data representative of the position of each sampling jar (3a, 3b, 3c) within the container (4), with which the cavity of the device (7) is equipped, are defined by means for detecting any shape, the cavity also being equipped with means for rotating the container (4), and the apparatus further comprising:
- computer processing means (18) which are connected to the device (7) by wired or wireless data transmission means (16) and integrate a software application which is executed to:
* store in memory means, for each jar (3a, 3b, 3c), in combination, the identification data (6a) that it comprises, the data (12a, 12b) representative of its position within the container (4), and data relating to the history of the biological sample (2) that it contains,
* detect the occurrence of a given event in the history of each jar (3a, 3b, 3c) and generate via a user interface (17) a list indicating the identification data (6a) of the jars (3a, 3b, 3c) for which the event is detected and the data (12a, 12b) representative of the position within the container (4) of the jars (3a, 3b, 3c) for which the event is detected.

2. Apparatus (1) according to claim 1, **characterized in that** the means for rotating the container (4) are defined by a shaft (13) rotatably mounted in the cavity (8) of the device (7), while the container (4) consists of a tray having a lower face (40) and an upper face (41) comprising the recesses (5a, 5b, 5c), the shaft (13) and the tray (4) having means for temporarily assembling the tray (4) to the shaft (13), which means are designed to prevent rotation of the tray (4) relative to the shaft (13).

3. Apparatus (1) according to claim 2, **characterized in that** the assembly means comprise an end piece (14) having a polygonal cross-section, which end piece extends from a free end of the shaft (13), and an orifice (15) which is complementary in shape to the cross-section of the end piece (14) and which extends from the lower face (40) of the tray (4) through at least part of the thickness of the tray.

4. Apparatus (1) according to any of the preceding claims,
**characterized in that** the recesses (5a, 5b, 5c) of the container (4) have shapes and dimensions that vary from one another.

5. Apparatus (1) according to any of claims 1 to 4, **characterized in that** the recesses (5a, 5b, 5c) and/or the sampling jars (3a, 3b, 3c) comprise foolproofing means to ensure appropriate positioning of a jar (3a, 3b, 3c) in a recess (5a, 5b, 5c).

6. Apparatus (1) according to any of the preceding claims,
**characterized in that** it comprises an additional encoded data reader (9) connected to the computer processing means (18).

7. Apparatus (1) according to any of the preceding claims,
**characterized in that** the container (4) comprises means for signaling the position of jars (3a, 3b, 3c) for which a given event is detected, the signaling means being connected to the computer processing means.

8. Apparatus (1) according to any of the preceding claims,
**characterized in that** it comprises means for automatically extracting, from the container (4), jars (3a, 3b, 3c) for which a given event is detected, which means are connected to the computer processing means.

9. Apparatus (1) according to any of the preceding claims,
**characterized in that** it comprises at least one furniture element designed to house a plurality of containers (4).

10. Method for the automated management of a plurality of biological samples (2) each placed in a sampling jar (3a, 3b, 3c) comprising a base (30) on which a peripheral wall (31) is mounted and which is closed by a lid (32) by means of the apparatus (1) according to any of the preceding claims, in which the following steps are carried out:
- a plurality of sampling jars (3a, 3b, 3c) that have identification data (6a) affixed to their peripheral wall (31) and/or lid (32) are placed in the recesses (5a, 5b, 5c) of at least one container (4),
- for each jar (3a, 3b, 3c) placed in the container (4), the identification data (6a), the data (12a, 12b) representative of its position within the container (4) and data relating to the history of the biological sample (2) it contains are collected and stored by means of the device (7) and the computer processing means (18) in combination,
- the software application contained in the computer processing means (18) is set so as to detect the occurrence of a given event in the data relating to the history of the biological sample (2) contained in each jar (3a, 3b, 3c),
- the user interface (17) automatically displays a list indicating the identification data (6a) of the jars (3a, 3b, 3c) for which the given event is detected and the data representative of the position within the container (4) of each sampling jar (3a, 3b, 3c) for which the given event is detected,
- the jars (3a, 3b, 3c) for which the given event is detected are extracted from the container (4) and destroyed, **characterized in that,** in order to collect the data representative of the position of each sampling jar (3a, 3b, 3c) within the container (4), recessed or raised shapes (12a, 12b) in the vicinity of each recess (5a, 5b, 5c) which define the data representative of the position of each sampling jar (3a, 3b, 3c) within the container (4) are detected, and means are used to detect any recessed or raised shape in the immediate vicinity of each recess.

11. Method according to the preceding claim, **characterized in that** when the given event is detected, means for signaling the position of the jars (3a, 3b, 3c) to be extracted, which means the container (4) comprises, are automatically triggered.

12. Method according to any of claims 10 or 11, **characterized in that** when the given event is detected, means for automatically extracting the jars (3a, 3b, 3c) from the receptacle (4), which means the apparatus (1) comprises, are triggered. ]
